# EUROPEAN PATENT APPLICATION

(11) **EP 4 257 058 A1**
(43) Date of publication of application: **11.10.2023**
(21) Application number: 22382322.0
(22) Date of filing: 04.04.2022
(51) Int. Cl.: A61B 8/10, A61B 8/00, A61B 8/08

(54) **TRANSDUCER, SYSTEM AND METHOD FOR OBTAINING BIOMECHANICAL PROPERTIES OF OCULAR TISSUE**

(71) Applicant: Consejo Superior de Investigaciones Cientificas, 28006 Madrid (ES); The University of Rochester, Rochester, New York 14642 (US); University of Houston System, Houston, Texas 77004 (US)
(72) Inventor: ZVIETCOVICH ZEGARRA, José Fernando, Madrid (ES); MARCOS CELESTINO, Susana, Rochester (US); LARIN, Kirill, Houston (US); MONTERO DE ESPINOSA, Francisco, Madrid (ES)
(74) Representative: Elion IP, S.L.

(57) **Abstract**

An ultrasonic transducer (10) for generating an ultrasonic wave (100) comprising:
- a sound emitting surface (12) having a shape of a spherical segment with a sphere radius (13);
- the sound emitting surface (12) having a through hole (11) coinciding with the first segment base having smaller first radius (r1) than the second radius (r2) of the second segment base;
- the ultrasonic wave generated by the sound emitting surface (12) is a focused ultrasonic beam which, at a focal point (14), produces a beam spot (101) having:
- an axial dimension (111) along an axis of symmetry of the spherical segment between 0.50 mm and 1.1 mm; and
- a lateral dimension (112) perpendicular to the axis of symmetry of the spherical segment between 0.4 mm and 1.2 mm;

- the ultrasonic wave has a frequency in the range from 300 kHz to 700 kHz; and in that
- a distance between the second segment base and the focal point is between 24 mm to 64 mm.

A system and a method for measuring biomechanical properties of ocular tissue are also disclosed.

## Description

### TECHNICAL FIELD

The present invention generally relates to the field of ophthalmology. More specifically, the present invention relates to a non-contact technique for obtaining biomechanical parameters of ocular tissue.

### STATE OF THE ART

Several studies have demonstrated the importance of measuring ocular biomechanics and obtaining certain biomechanical parameters. Among others, these biomechanical parameters can be used for diagnosing diseases at an early stage, also for monitoring treatments, and for developing tools for decision-making before a surgical/treatment procedure. In particular, keratoconus is a degenerative disease characterized by the thinning and loss of rigidity of the cornea. It occurs in approximately one in 375 individuals in developing countries, and it is the leading cause of corneal transplantation in the USA. For ophthalmologists, detecting keratoconus in sub-clinical stages is fundamental for early treatment planning and preventing further degeneration of cornea. The standard clinical diagnosis of keratoconus includes corneal topography (to detect irregular corneal curvature and cone formation), pachymetry (to detect thin regions of corneal thickness), and, still in experimental phases, biomechanics (to detect abnormal loss of rigidity of the cornea).

Oculus Incorporation (Wetzlar, Germany) developed and implemented commercial and clinically available equipment to provide biomechanical information of the cornea. This technology is based on the macro-deformation of the cornea produced by the release of an air-puff stimulus. The main problems with this technology are the following: (1) it only provides a single biomechanical parameter that represents the whole cornea extension (in most cases in only one meridian), preventing the localization of abnormally softer regions in the cornea in early stages of keratoconus; and (2) the biomechanical parameter provided is based on air-puff corneal macro-deformation which is coupled with other variables such as intraocular pressure, corneal thickness, and corneal elasticity. This makes difficult the interpretation of the provided biomarker in terms of corneal stiffness and the separation of normal from a pathological corneal elasticity, including the grading of different stages of pathology.

Optical coherence elastography (OCE) based on the propagation of mechanical waves is the current research trend for probing viscoelastic properties of soft tissues including the ocular tunic. This technique in formed by three main components: (1) a mechanical excitation module that stimulates the tissue in order to produce mechanical wave propagation; (2) a phase-sensitive optical coherence tomography (OCT) system (or any other optical technology capable of measuring motion at the nano-meter range) that measures the motion (displacement or particle velocity) produced by the propagation of mechanical waves in the tissue; and (3) a post-processing approach to estimate mechanical wave properties (e.g. speed and attenuation) from the acquired data to infer viscoelastic properties of tissues (e.g. Young's and shear moduli) using the appropriate wave models (e.g. shear or Lamb wave propagation model) and the support of numerical simulations. Patent document WO 2016/077173 A1 discloses a system using an excitation force and phase-sensitive optical coherence elastography (OCE), in conjunction with a data analyzing algorithm, for measuring and quantifying biomechanical parameters of tissues in situ and *in vivo.*

Due to the potential of OCE in the study of corneal biomechanics, the research has continued evolving during the years including the development of non-contact mechanical excitation methods more suitable to clinical application. The use of air-coupled ultrasonic (ACUS) transducers to generate non-contact excitation and wave propagation in OCE applications has been proposed, for example, in patent document WO 2017/205809 A1.

Most of the studies presented, up to this point, have been conducted at *ex-vivo* level and only some studies have been conducted *in-vivo*, in animal models.

The fact is that the existing non-contact systems for generation and detection of a mechanical wave in a soft tissue, fail to produce detectable mechanical excitation in human corneas. Accordingly, new and/or improved systems and methods for mapping elastic properties of a soft tissue remain of interest.

### SUMMARY

The present disclosure intends to solve the shortcomings of prior-art devices and methods by providing a transducer, a system and a method capable for the robust generation motion and wave propagation in human corneas, which are then capable of mapping biomechanical properties of ocular tissue with high precision and reliability.

A first aspect relates to an ultrasonic transducer for generating an ultrasonic wave, the ultrasonic transducer comprising:
- a sound emitting surface having a shape of a spherical segment, defined by a sphere radius, first and second segment bases having respective first and second radii and a sphere segment height;
- the sound emitting surface has a through hole coinciding with the first segment base having smaller radius than the radius of the second segment base;
- the ultrasonic wave generated by the sound emitting surface is a focused ultrasonic beam which, at a focal point, produces a beam spot having:
   - an axial dimension (or depth of field, DOF) along an axis of symmetry of the spherical segment between 0.50 mm and 1.1 mm; and
   - a lateral dimension (or focal beam waist, FBW) perpendicular to the axis of symmetry of the spherical segment between 0.4 mm and 1.2 mm;
- the ultrasonic wave generated by the sound emitting surface has a frequency in the range from 300 kHz to 700 kHz; and
- a distance between the second segment base and the focal point is between 24 mm to 64 mm.

The ultrasonic transducer as just defined is suitable for the study of human corneas. It can be used for the development of biomarkers to characterize sub-clinical stages and/or progression of keratoconus and other ocular diseases.

The beam spot produced by the ultrasonic transducer can be measured at full width at half maximum (FWHM). In certain embodiments the axial dimension or depth of field of the beam spot is in the range of 0.75 mm and 0.90 mm, and the lateral dimension or focal beam waist is around 0.6 mm. A beam spot with these dimensions has proved to provide better mechanical excitation of a human cornea, in terms of the mechanical wave propagated through the cornea.

The distance between the second segment base and the focal point (that is, the working distance) is to be understood as the shortest distance between the focal point and a plane containing the second segment base.

The ultrasonic wave generated by the sound emitting surface can have a frequency of 450 kHz to 550 kHz, which has proven to be an optimum frequency range for providing measurable displacement at the ocular tissues.

In certain embodiments of the ultrasonic transducer, the sphere radius is in the range from 30 mm to 75 mm; and the radius of the second segment base is between 17.5 mm and 40 mm. Providing an ultrasonic transducer with these dimensions enables the generation of the beam spot at a distance of more than 24 mm, thus making the ultrasonic transducer suitable for human applications.

In certain embodiments the ultrasonic transducer comprises an array of electrodes distributed around the sound emitting surface, thereby providing 3D beam steering. Additionally, this configuration can be easier to manufacture than an array of single ultrasonic transducers. The array of electrodes can comprise polygonal and/or elliptical electrodes, which are distributed over the sound emitting surface in a symmetric manner.

In certain embodiments the electrodes of the array of electrodes are annular or ring-shaped and are distributed over the sound emitting surface in a concentric manner. This configuration allows provides better control of beam depth.

The ultrasonic transducer may comprise an array of ultrasonic transducers, which are arranged to form the sound emitting surface. As in the case of having multiple electrodes, having an array of several transducers provides control of 3D beam steering, and may also result in easier manufacturing process.

The ultrasonic transducer as defined in the foregoing can be intended for *in vivo* non-contact elastography of the human eye, particularly in clinical environments.

In some embodiments the radius of first segment base of the ultrasonic transducer is between 6.5 mm and 8.5 mm; particularly, around 7.5 mm allows measuring the full human cornea (whose average diameter is around 12 mm) and include some sclera (diameter around 15 mm).

The sound emitting surface of the ultrasonic transducer is capable of irradiating ultrasound, and the ultrasonic wave generated by the sound generating surface is focused on the center of the sphere corresponding to the sound emitting surface, that is, at the focal point. The sound emitting surface -be it in the form of a single transducer or of an array of transducers- is preferably made of a piezoelectric material, such as ceramic or composite material.

In certain embodiments the sound emitting surface of the ultrasonic transducer comprises a coating layer, such as a porous membrane filter. This coating layer provides a matching between acoustic impedances of the air and the transducer electrode coupling at around the resonance frequency of the ultrasonic transducer.

In the present disclosure, the ocular tissue comprises the ocular tunic, particularly, the cornea and the sclera.

Another aspect of the disclosure relates to a system for measuring biomechanical properties of ocular tissue, the system comprising:
- an ultrasonic transducer as defined in the foregoing, which ultrasonic transducer is configured for delivering a focused ultrasonic beam onto an eye of a user;
- an imaging device operatively coupled to the ultrasonic transducer, and arranged in a confocal manner with the ultrasonic transducer; the imaging device being configured to provide measurements of locations and displacements of a mechanical wave in the ocular tissue produced by the focused ultrasonic beam; and,
- a processor for processing the plurality of measurements provided by the imaging device to provide measurements of propagation of the mechanical wave (which may include speed, attenuation and/or dispersion) and thickness of the ocular tissue.

The system as just defined proposes a new design of a confocal ultrasonic transducer for the study of human ocular tissue (particularly suitable for corneas), for providing biomechanical properties of the excited -by means of ultrasound- ocular tissue. One or more of the following parameters of the ocular tissue can be obtained: shear modulus, Young's modulus, viscoelasticity, non-linearity and anisotropy.

In certain embodiments the system is further configured to develop biomarkers to characterize sub-clinical stages and/or progression of keratoconus and other ocular diseases. According to certain embodiments, the processor is further configured to provide one or more of the following biomarkers: anisotropy of wave speed (SAWS) and a thickness-speed map (TSM).

The imaging device can be any device suitable for providing measurements of locations and displacements, such as an OCT, which can be configured to work in phase-sensitive mode.

In the present, operatively coupled means that excitation of the ultrasonic transducer and acquisition at the imaging device are coordinated, usually synchronize.

In certain embodiments the imaging device is configured to acquire measurements at multiple meridians, thus, enabling that the measurements are taken covering full 360°. The system is thus capable of producing measurements in a polar plot.

In certain embodiments the system is configured for acquiring in-vivo biomechanical parameters for tracking ocular treatments and/or for screening ocular diseases and/or for classifying different keratoconus stages and being able to detect keratoconus in subclinical stages.

It should be noted that in the above embodiments the processor can be implemented as a separate element. But it is also possible that the processor and the imaging device are implemented in a single device, such as an OCT configurable to carry out additional post-processing.

Another aspect of the invention relates to a method for obtaining biomechanical properties of ocular tissue, the method comprising:
- generating an ultrasonic wave having a resonant frequency in the range from 300 kHz to 700 kHz;
- delivering the ultrasonic wave onto ocular tissue in a confocal manner with an imaging device;
- capturing with the imaging device measurements of locations and displacements of a mechanical wave in the ocular tissue produced by ultrasonic wave delivered onto the ocular tissue; and,
- processing the measurements of locations and displacements provided by the imagine device to provide measurements of propagation of the mechanical wave and, preferably, thickness of the ocular tissue.

According to certain embodiments, the ultrasonic wave is generated by an ultrasonic transducer as defined in respect with the first aspect of the present disclosure. And the second segment base of such ultrasonic transducer can be arranged at the working distance of the ultrasonic transducer, which is between 24 mm to 64 mm from the eye of the user.

In the system and/or the method disclosed herein, the ocular tissue can be a cornea or a sclera of a human eye.

The different aspects and embodiments of the invention defined in the foregoing can be combined with one another, as long as they are compatible with each other.

Additional advantages and features of the invention will become apparent from the detailed description that follows and will be particularly pointed out in the appended claims.

### BRIEF DESCRIPTION OF THE DRAWINGS

To complete the description and in order to provide for a better understanding of the invention, a set of drawings is provided. Said drawings form an integral part of the description and illustrate embodiments of the invention, which should not be interpreted as restricting the scope of the invention, but just as examples of how the invention can be carried out. The drawings comprise the following figures:
Figures 1 and 2 schematically shows top and bottom perspective views, respectively, of an embodiment of ultrasonic transducer.
Figure 3 shows a cross section of the ultrasonic transducer shown in Figure 1.
Figure 4 shows a system for obtaining biomechanical properties of an eye, including an ultrasonic transducer and an imaging device.
Figure 5 is an enlarged view of the ultrasonic beam spot produced by the ultrasonic transducer.
Figure 6 shows an array of ring-shaped ultrasonic transducers, arranged concentrically.
Figure 7 shows another embodiment of an array of disc-shaped ultrasonic transducers.
Figure 8 shows an ultrasonic transducer having an array of ring-shaped electrodes. And
Figure 9 shown an ultrasonic transducer having an array of circular electrodes.
Figures 10 and 11 show thickness-speed maps of normal and keratoconus human corneas, respectively.
Figures 12 and 13 show meridional-dependent wave speed and spatial anisotropy of wave speed (SAWS) of normal and keratoconus human corneas, respectively.

### DESCRIPTION OF WAYS OF CARRYING OUT THE INVENTION

The following description is not to be taken in a limiting sense but is given solely for the purpose of describing the broad principles of the invention. Embodiments of the apparatus and method of the present disclosure will be described by way of example, with reference to the accompanying drawings.

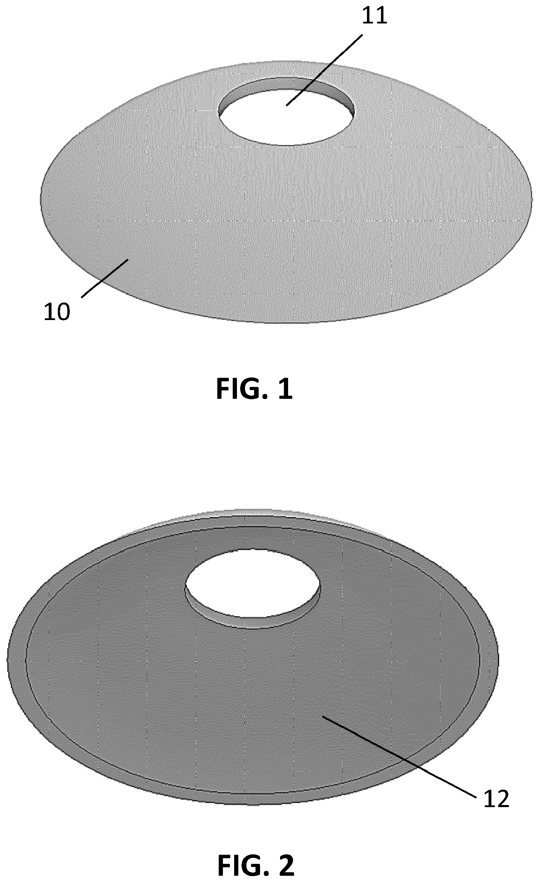

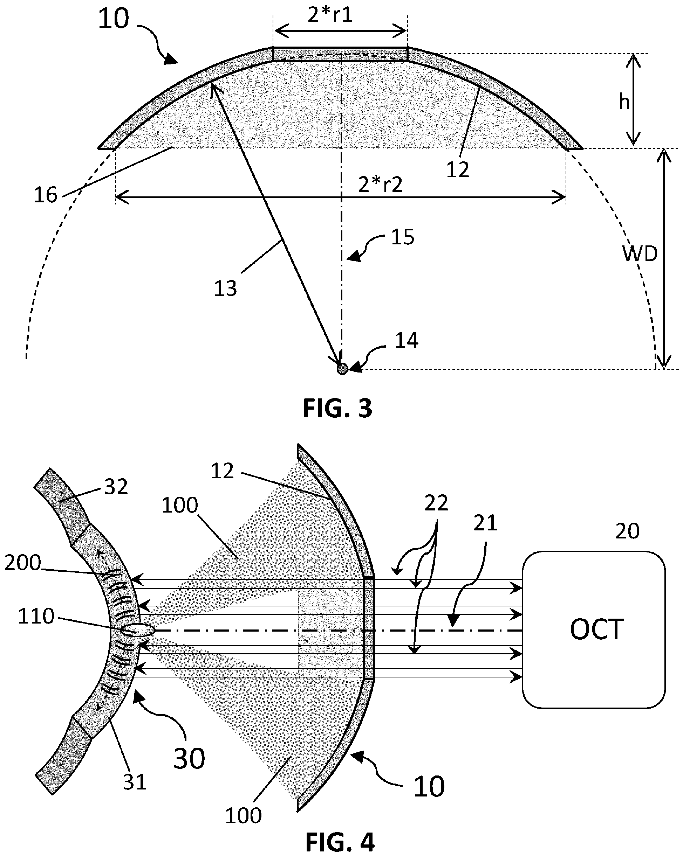

According to the present disclosure, a 500 kHz ultrasonic air-coupled transducer 10 (as the one shown in Figures 1-2) co-focused with an optical coherence tomography device 20 was used to generate quasi-harmonic mechanical perturbation at the corneal apex of a human eye 30. A schematic representation of such a system is shown in Figure 4, which shows the details and location of the ultrasonic transducer 10 with respect the OCT device 20 and the subject's cornea 31.

Figure 1 shows a perspective top view of an ultrasonic transducer 10 for generating an ultrasonic wave 100. The ultrasonic transducer 10 shown in Figure 1 has the shape of a spherical segment with a through hole 11 coincident with the smaller base of the spherical segment. The ultrasonic transducer 10 has a sound emitting surface 12 for irradiating ultrasound, which is spherical, defined by a sphere radius 13, which is a focal distance of the ultrasonic transducer, defining a focal point 14. Reference number 15 represents the axis of the ultrasonic transducer 10, and of the corresponding ultrasound wave 100.

The smaller segment base of the spherical segment -coincident with the through hole 11- has a first radius r1, and the bigger segment base of the spherical segment has a second radius r2. The ultrasonic transducer has a sphere segment height h.

In the embodiment shown, the internal surface of the ultrasonic transducer 10 is covered with a matching layer (not represented) for air coupling at 0.5 MHz. The thickness of the one-piece ultrasonic transducer shown in Figure 1 is of 4.5 mm approximately.

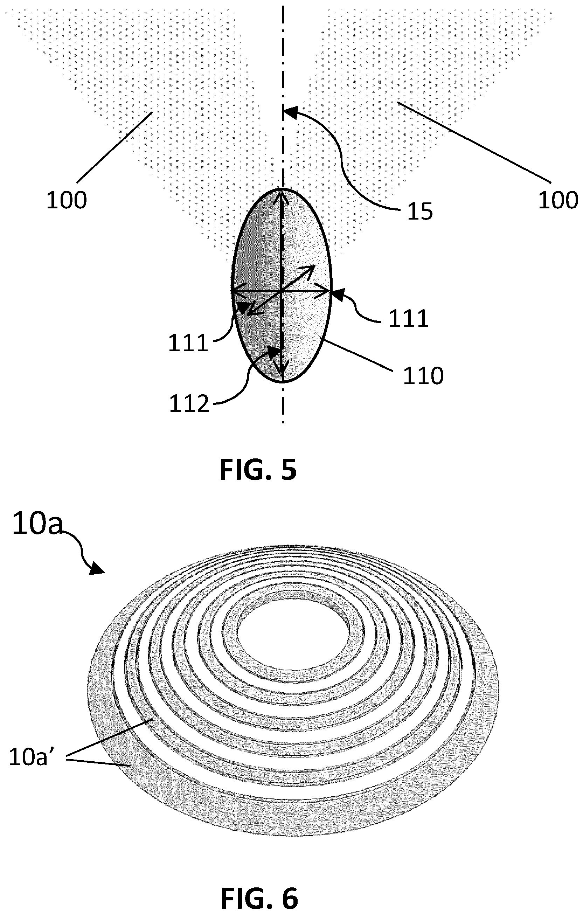

As shown in Figures 3 and 4, the ultrasonic transducer 10 generates an ultrasound wave 100, which is a focused ultrasonic beam which, at the focal point 14, produces an acoustic spot 110. The acoustic spot 110 is schematically represented in Figure 5. The spatial properties of the focused acoustic spot 101 along the lateral direction 111 and axial direction 112 are the following: focal beam waist of around 1 mm, and depth of field of around 0.85 mm, respectively. The focused acoustic spot 110 is located at a working distance WD of 24.5 mm from the ultrasonic transducer aperture 16 (which coincides with the bigger segment base, having radius r2), where an apex of a cornea 31 of a human eye is located. This acoustic spot 110 produces a mechanical wave 200, which propagates from the corneal apex towards the sclera 32.

The proposed design allows for the robust excitation of motion and wave propagation in human cornea since:
- It allows for the easy accommodation of the eye and face of the patient in front of the transducer due to the extended working distance, around 24.5 mm.
- It produces at least 2 times more displacement than previously reported designs using an ultrasonic transducer (e.g. from around 0.5 µm to around 1 µm).
- It allows for the axial movement of the eye within the acoustic depth of field of -0.85 mm (-1.7 times the corneal thickness) for proper wave excitation.

The non-contact ultrasonic wave-base optical coherence elastography system schematically shown in Figure 4 was used to retrieve mechanical parameters of *in-vivo* human cornea in 3D and used as a diagnostic tool to discriminate normal corneas from early sub-clinical and different advanced stages of keratoconus and corneas with ectasia. This system and methodology overcome the drawbacks of existing solutions, since:
- It measures biomechanical parameters related to corneal shear modulus which is an intrinsic biomechanical parameter related to corneal shape integrity. Shear modulus is hypothesized to change before topographic and pachymetry changes that are manifested in keratoconus.
- The biomechanical parameters related to shear modulus are provided in multiple corneal meridians which allows localization of corneal regions with abnormal stiffness. This resource further enables not only the localization of the keratoconus, but also guide doctors in the application of localized treatment.
- The grading nature of shear modulus estimated at all corneal meridians can be used not only to localize sub-clinical keratoconus, but to classify disease stages.

The system shown in Figure 4 produces quasi-harmonic excitation at the apex of the cornea (and/or other ocular tissues) in the frequency range of 0.4 to 10 kHz in order to produce mechanical wave propagation (shear waves, Lamb waves, and any other surface acoustic wave). The OCT device 20 captures the motion produced in the cornea by the wave propagation phenomenon.

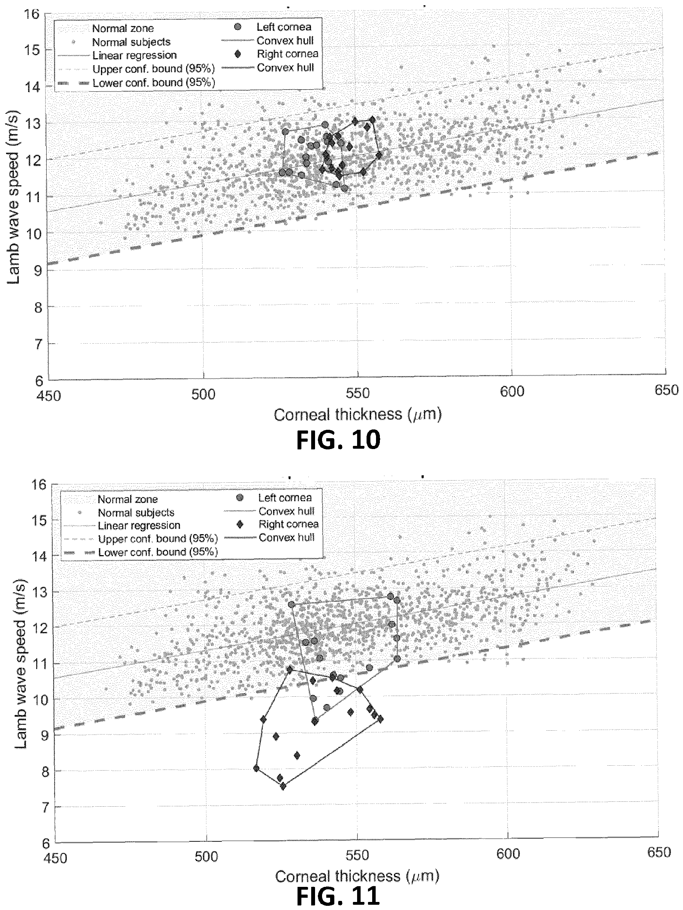

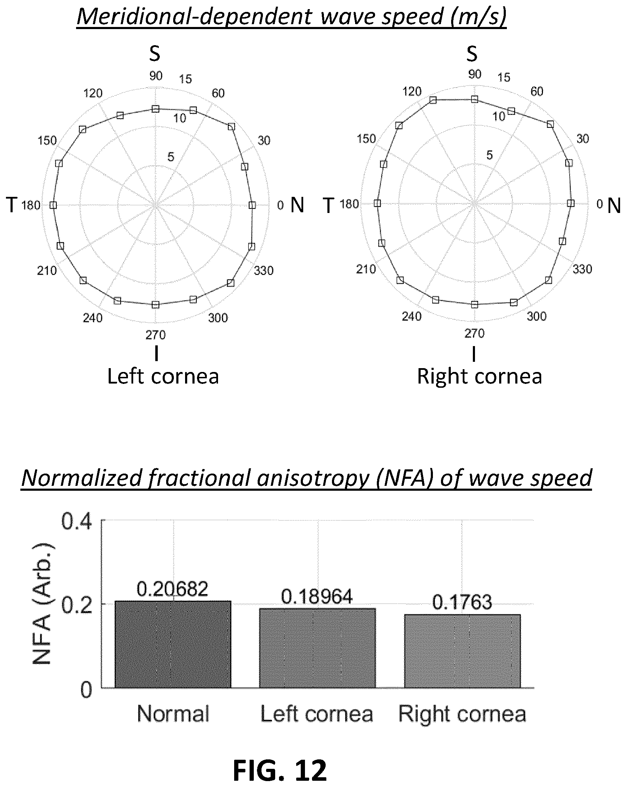

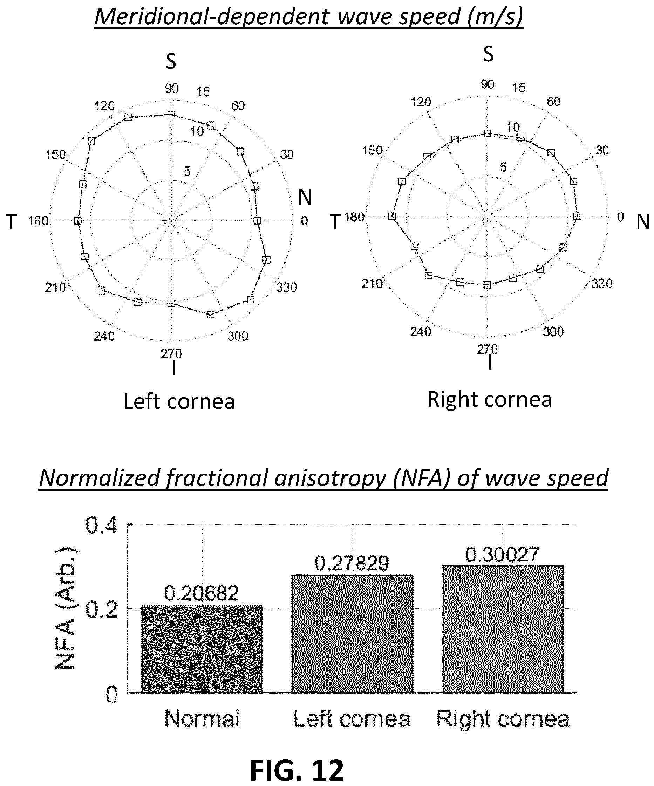

A software package processes the signals obtained with the OCT device 20 and provides measurements of wave propagation speed and average corneal thickness within multiple corneal meridians covering 360°. Specifically, two biomarkers capable to classify different stages of keratoconus are provided:
- A thickness-speed map (TSM), where measured corneal values (i.e. speed and thickness) for each corneal meridian are projected to separate normal (as shown in Figure 10) from pathological cases, as shown in Figure 11, with clear differentiation of a sub-clinical and stage III keratoconus corneas. In the TSM, the left cornea presents four meridians in the pathological zone, while the right cornea has thirteen meridians in the pathological zone.
- Spatial anisotropy of wave speed (SAWS) was calculated from the meridional-dependent wave speed of each cornea (left and right). Measurements of speed and thickness were calculated in the following formats: meridional-dependent wave speed polar plot (shown in the top images if Figures 12 and 13), and spatial anisotropy of wave speed. The normalized fractional anisotropy (NFA) was calculated from these two data to compare with individual cases. The NFA is represented in the bottom images of Figures 12 and 13. Figure 12 shows the biomarkers for a normal eye, and Figure 13 for sub-clinical stage (left cornea) and stage III keratoconus (right corneas) corneas.

Lamb wave propagation speed and average thickness were measured within sixteen corneal meridians covering 360 degrees during 1 s of acquisition. Measurements were conducted in forty-two human healthy subjects (control group, N = 84 corneas; age: 20-50; corneal astigmatism <2 diopters) and three keratoconic patients (N = 5 corneas; age: 14-50). All measurements were fully non-contact, and followed the approved research protocol adhered to the tenets of the Declaration of Helsinki. Spatial anisotropy of wave speed (SAWS) was calculated from the meridional-dependent wave speed of each cornea. Speed and thickness for each corneal meridian were projected in a thickness-speed map (TSM) for further statistical analysis.

### Results of the study

SAWS was statistically significantly higher in advanced (0.353, p < 0.001, N = 3) and pre-clinical (0.249, p = 0.04, N = 2) keratoconus corneas when compared to the baseline. Moreover, we found a linear correlation between meridional speed and thickness (RMSE = 0.738, p < 0.001) in normal corneas when measurements were projected into the TSM. A 95% confidence level was used as a baseline metric to separate normal (stiffer) from abnormal (softer) corneal elasticity. Abnormal elasticity was found in at least 10 out of 16 meridians in advanced keratoconus corneas (p < 0.001, N = 3), and in at least in 4 out of 16 meridians in pre-clinical keratoconus corneas (p < 0.001, N = 2).

The results show important biomechanical differences in SAWS and TSM between normal and keratoconus corneas, suggesting those as potential biomarkers for progression and severity of keratoconus, following sensitivity and specificity studies on a larger sample of keratoconic (and their contralateral) eyes.

Figures 6-9 show other possible embodiments of the ultrasonic transducer:
- In Fig. 6 the ultrasonic transducer 10a is made of an array of ring-shaped ultrasonic transducers 10, arranged concentrically.

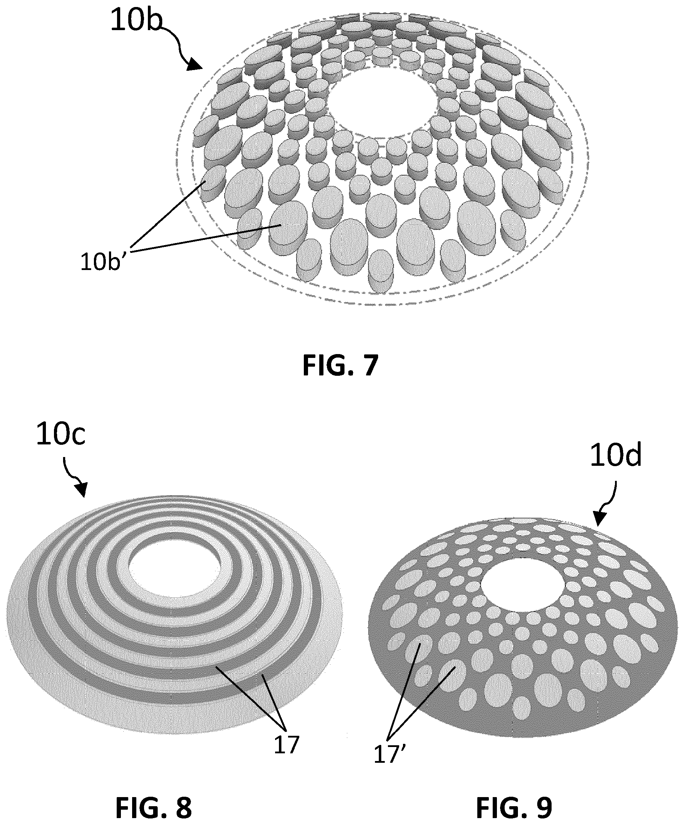

- In Fig. 7 the ultrasonic transducer 10b is made of an array of disc-shaped ultrasonic transducers.
- In Fig. 8 the ultrasonic transducer 10c has an array of ring-shaped electrodes 17.
- And in Fig. 9 the ultrasonic transducer 10d has an array of circular electrodes 17'.

In this text, the terms first, second, third, etc. have been used herein to describe several devices, elements or parameters, it will be understood that the devices, elements or parameters should not be limited by these terms since the terms are only used to distinguish one device, element or parameter from another. For example, the first support could as well be named second support and vice versa, without departing from the scope of this disclosure.

In this text, the term "comprises" and its derivations (such as "comprising", etc.) should not be understood in an excluding sense, that is, these terms should not be interpreted as excluding the possibility that what is described and defined may include further elements, steps, etc. On the other hand, the invention is obviously not limited to the specific embodiment(s) described herein, but also encompasses any variations that may be considered by any person skilled in the art (for example, as regards the choice of materials, dimensions, components, configuration, etc.), within the general scope of the invention as defined in the claims.

## Claims

1. An ultrasonic transducer (10) for generating an ultrasonic wave (100), the ultrasonic transducer (10) comprising:
- a sound emitting surface (12) having a shape of a spherical segment, defined by a sphere radius (13), first and second segment bases having respective first and second radii (r1, r2) and a sphere segment height (h);
- the sound emitting surface (12) having a through hole (11) coinciding with the first segment base having smaller first radius (r1) than the second radius (r2) of the second segment base,
**characterised in that**:
- the ultrasonic wave generated by the sound emitting surface (12) is a focused ultrasonic beam which, at a focal point (14), produces a beam spot (101) having:
- an axial dimension (111) along an axis of symmetry of the spherical segment between 0.50 mm and 1.1 mm; and
- a lateral dimension (112) perpendicular to the axis of symmetry of the spherical segment between 0.4 mm and 1.2 mm;
- the ultrasonic wave has a frequency in the range from 300 kHz to 700 kHz; and **in that**
- a distance between the second segment base and the focal point is between 24 mm to 64 mm.

2. The ultrasonic transducer (10) of claim 1, wherein:
- the sphere radius (13) is in the range from 30 mm to 75 mm; and
- the radius (r2) of the second segment base is between 17.5 mm and 40 mm.

3. The ultrasonic transducer (10, 10c, 10d) of any previous claim, wherein the ultrasonic transducer comprises an array of electrodes (17, 17') distributed over the sound emitting surface (12).

4. The ultrasonic transducer (10c) of claim 3, wherein the plurality of electrodes (17) are ring-shaped and are distributed over the sound emitting surface in a concentric manner.

5. The ultrasonic transducer (10, 10a, 10b) of any previous claim, wherein the ultrasonic transducer comprises an array of ultrasonic transducers (10a, 10b) forming the sound emitting surface (12).

6. The ultrasonic transducer (10) of any previous claim, wherein the ultrasonic transducer is intended for *in vivo* non-contact elastography of the human eye (30).

7. The ultrasonic transducer (10) of any previous claim, wherein the radius (r1) of first segment base is between 6.5 mm and 8.5 mm.

8. A system for measuring biomechanical properties of ocular tissue comprising:
- an ultrasonic transducer (10) as defined in any previous claim, the ultrasonic transducer being configured for delivering a focused ultrasonic beam onto an eye (30) of a user;
- an imaging device (20) operatively coupled to the ultrasonic transducer (10) and arranged in a confocal manner with the ultrasonic transducer; the imaging device (20) being configured to provide measurements of locations and displacements of a mechanical wave (200) in the ocular tissue produced by the focused ultrasonic beam (110); and,
- a processor for processing the measurements provided by the imaging device (10) to provide measurements of propagation of the mechanical wave and, preferably, thickness of the ocular tissue.

9. The system of claim 8, wherein the processor is further configured for obtaining one or more of the following parameters: shear modulus, Young's modulus, viscoelasticity, non-linearity and anisotropy.

10. The system of any of claims 8-9, wherein the measurements are acquired at multiple meridians covering 360°.

11. The system of any of 8-10, wherein the processor is further configured to provide one or more of the following biomarkers: anisotropy of wave speed (SAWS) and a thickness-speed map (TSM).

12. The system of any of claims 8-11, configured for acquiring in-vivo biomechanical parameters for tracking ocular treatments and/or for screening ocular diseases and/or for classifying different keratoconus stages.

13. Method for obtaining biomechanical properties of ocular tissue, the method comprising:
- generating an ultrasonic wave (100) having a resonant frequency in the range from 300 kHz to 700 kHz;
- delivering the ultrasonic wave onto ocular tissue (31, 32) in a confocal manner with an imaging device (20);
- capturing with the imaging device (20) measurements of locations and displacements of a mechanical wave (200) in the ocular tissue produced by ultrasonic wave (100);
- processing the measurements of locations and displacements provided by the imagine device to provide measurements of propagation of the mechanical wave and, preferably, thickness of the ocular tissue.

14. The method of claim 13, wherein the ultrasonic wave is generated by the ultrasonic transducer of any of claims 1-7.

15. The method of claim 14, wherein the second segment base (16) of the ultrasonic transducer (10) is arranged at a distance of between 24 mm to 64 mm from the eye of the user.
